# EUROPEAN PATENT SPECIFICATION

(11) **EP 3 348 292 B1**
(45) Date of publication and mention of the grant of the patent: **17.02.2021**
(21) Application number: 15903564.1
(22) Date of filing: 08.09.2015
(51) Int. Cl.: A61M 5/14, A61J 1/10, A61M 39/10, A61M 39/26, A61M 39/28, A61M 5/168, A61M 39/22, A61M 5/162

(54) **INFUSION SET**
INFUSIONSSET
ENSEMBLE POUR PERFUSION

(43) Date of publication of application: 18.07.2018
(73) Proprietor: Kobayashi & Co., Ltd., Taito-ku, Tokyo 111-8620 (JP)
(72) Inventor: FUKUOKA, Koji, Kobe-shi Hyogo 650-0047 (JP); KAWAOI, Go, Kobe-shi Hyogo 650-0047 (JP); ENDO, Imari, Kobe-shi Hyogo 650-0047 (JP); MITSUHASHI, Masaaki, Kobe-shi Hyogo 650-0047 (JP)
(74) Representative: V.O.
(86) International application number: PCT/JP2015/075523
(87) International publication number: WO 2017/042899

(56) References cited:
- EP-A1- 2 881 138
- WO-A1-2010/001939
- JP-A- H06 105 890
- JP-A- H10 108 908
- JP-A- 2003 320 029
- JP-A- 2003 509 163
- JP-A- 2004 267 377
- JP-A- 2010 018 539
- JP-A- 2014 200 415
- JP-U- H0 341 454
- US-A1- 2011 132 482
- US-A1- 2013 274 702

## Description

### TECHNICAL FIELD

The present invention relates to an infusion set, and to an infusion set sealed within a package, which, by making it possible for an infusion container to be pierced on a first attempt, permit priming operations to be carried out separately at respective sets of main tubing and secondary tubing of an infusion set without contamination of areas thereabout, and which, by thereafter making it possible, at any necessary desired timing, for said set of secondary tubing at which the infusion container has been pierced to be made integral with and inseparable from the set of main tubing of the infusion set, permit an infusion to be administered in stable fashion without occurrence of contamination or misidentification of infusion administration procedures.

### BACKGROUND ART

Medical treatments have conventionally been carried out in which therapeutic medications are formulated as infusions which are administered intravenously. Infusions employing therapeutic medications in the form of anticancer agents, nutrients, and the like must typically be administered in high dosages. Furthermore, where a plurality of medications are combined, each infusion must be administered in order, and the total administration dosage is quite high. On the other hand, as administration of infusions continues, because a sudden rise in the concentration of a drug within the blood increases the risk of occurrence of anaphylactic shock, cardiac arrhythmia, and other such side effects, there is a need for physicians and nurses to carefully continue to adjust infusion administration dose while monitoring the condition of the patient. However, carrying out administration by adjusting amount by means of injection is difficult, and as administration time goes on and the number of administrations increases, the patient experiences an increasing amount of bodily pain and is placed under an increasing amount of stress. For this reason, intravenous drip infusion is widely used as a technique for easily and continuously administering infusions to patients intravenously. During intravenous drip infusion, as medical equipment for causing a container having an infusion sealed therewithin to be connected to an intravenous drip needle that has been inserted within a blood vessel of a patient, an infusion set is employed, those in general use conventionally employing soft tubing to link the container having the infusion sealed therewithin with the intravenous drip needle, a mechanism employing a roller clamp and a drip chamber being provided midway along said soft tubing.

Before initiating intravenous drip infusion, physiological saline solution or other such infusion is made to pass through such an infusion set in advance, priming operations being carried out to adequately remove air in advance from the soft tubing, at which time it is necessary to pay adequate attention to ensure that air bubbles originating from such air are not allowed to enter the patient's blood vessel. However, with a conventional infusion set, even where caution is exercised there is a risk that an infusion may leak from the tip of the needle at the downstream-most end of the infusion set during priming, or that an accident could occur in which a hospital room or a hospital wing becomes contaminated, and so there has been a need for a strategy to prevent such accidents.

An infusion set has therefore been proposed that adopts a strategy to reduce the likelihood of leakage of dangerous drugs to the outside environment by splitting the region upstream of the drip chamber into multiple branches to establish two switchable priming flow paths, a shutoff clamp being arranged below the drip chamber and one of the priming flow paths being employed to carry out initial priming, and the priming flow path thereafter being switched, the shutoff clamp below the drip chamber being closed, and the other priming flow path thereafter being used to carry out priming of the remaining priming flow path (see Patent Reference No. 1).

However, while it may be true that the risk of contamination during initial priming is relatively low, the structure of the foregoing proposed infusion set is such that it permits leakage of the dangerous drug from below, and so from the standpoint of whether it adequately prevents leakage of dangerous drugs to the outside environment, there has been a need for further ingenuity. Furthermore, because the structure is such that terminal portions of the infusion set are open, operations have been complicated inasmuch as there has been a need to exercise care with respect to procedural discharge of physiological saline solution or other such solution used during priming from said terminal portions, and inasmuch as there has been a need for priming, which itself comprises multiple procedural steps, to be carried out twice, and so forth. In addition, even where adequate care is exercised with respect to procedural discharge of liquid, there is a risk of occurrence of damage to equipment and/or contamination of the hospital room interior as a result of unintentional spillage of the physiological saline solution that is discharged therefrom onto the infusion set, the stand, the equipment used to carry out intravenous drip infusion, and/or the floor of the hospital room.

Furthermore, with a conventional infusion set, where a plurality of anticancer agents were administered, it has been necessary to remove used infusion container(s) from the infusion set and replace these with different infusion container(s), and so it has been entirely possible for contamination to occur due to the small amount of liquid which remains in spike(s) and/or connector portion(s). It therefore being demanded that procedures be carried out with great care, the physician, nurse, and pharmacist responsible for carrying out procedures have been put under an extremely large amount of stress, as a result of which there has been a need for some further strategy.

Infusion sets are therefore in general use in which a plurality of sets of secondary tubing are arranged in advance so as to make it possible to use a mixing region and/or Y-tubing midway along the soft tubing to split it into multiple branches and make it possible to link a plurality of containers having infusions sealed therewithin. Where this is the case, it being necessary to cause this to remain as still as possible to prevent the infusion from leaking outside the blood vessel during intravenous drip infusion, the infusion set is suspended from an IV stand and is used at the patient's bedside, but because at least 3 to 5 hours is required for intravenous drip infusion, and there are cases in which extremely long times on the order of 48 hours may be required, the patient will have no choice but to at some point during the course thereof go to the toilet, eat a meal, or perform some other physiological activity. The patient must therefore, while continuing to receive the intravenous drip infusion with the intravenous drip needle still stuck in his or her arm, or after closing the roller clamp and reducing the drip rate, or temporarily shutting off and interrupting the drip, or the like, move together with the IV stand, linked to which is the infusion set from which the container having the infusion sealed therewithin is suspended.

However, particularly in conventional infusion sets such as those in which a plurality of sets of secondary tubing are arranged in advance, there has been a tendency for balance to become upset and there has been a risk of occurrence of accidents in which tubing at the plurality of sets of secondary tubing that have been arranged in advance gets caught on peripheral equipment and/or the physician, nurse, or pharmacist responsible for carrying out procedures and so forth. Furthermore, even where the patient, physician, nurse, and pharmacist have paid adequate attention with the intention of avoiding situations in which the infusion set tubing itself becomes twisted or tension acts thereon, there has been a tendency toward occurrence of pain at the location where the drip infusion is inserted and/or other such bodily pain on the part of the patient, and in situations where an intravenous drip needle inserted beneath the skin has moved, causing anticancer agent solution to leak outside the blood vessel, or further tension or forcible twisting has acted thereon, there has been an extremely large amount of stress during use, inasmuch as there has been a risk of occurrence of accidents in which the intravenous drip needle could become dislodged from the arm of the patient and/or the like. For this reason, there has been a need for some further strategy.

Furthermore, where a plurality of anticancer agents were administered in the form of infusions, particular attention has been required to avoid mixture of solutions for which mixture is contraindicated and to see that administration is carried out in the correct order. Because the type and number of therapeutic agents are different for every patient, infusion sets must be prepared that are reassembled in correspondence thereto. However, it is often the case that the containers employed for infusions have similar external appearance. For this reason, mixups with respect to the order in which spikes pierce containers, confusion as to which infusion line should be used for different anticancer agent solutions, mistaken order of administration, and other such accidents can easily occur. Efforts have therefore been made to prevent accidents due to human error on the part of physician, nurse, and/or pharmacist who use the infusion set and adjust infusions and anticancer agent solutions by applying labels to the various containers at the time that the infusions are prepared, attaching written warnings, and so forth to clearly specify administration procedure, and to make the various containers recognizable, to establish standard procedures for use specific to each of the various infusion sets with their many different constitutions, to publicize this in advance, and carry out training and so forth, but the situation remains unchanged and misidentification by the operator can still occur, as a result of which there has been a need for improvement.

### PRIOR ART REFERENCES

### PATENT REFERENCES

Patent Reference No. 1: International Patent Application Publication No. 2014/021390

US2011/132482 discloses a connector including an inlet port into which liquid is poured and which has a first flow path for the liquid, a liquid discharge port to which a tube body is connectable and which has a second flow path discharging liquid from the first flow path to the connected tube body, a gas discharge which discharges air in the first flow path, and a blocking device which, when the liquid is poured in the first flow path, temporarily stops the liquid flow. Use of the connector involves sealing an opening section of the liquid discharge port, pouring liquid in the inlet port, causing air in the first flow path to be discharged, temporarily stopping the flow of the liquid in the first flow path, unsealing the opening section of the liquid discharge port, connecting the tube body to the liquid discharge port, and resuming flow of the liquid.

EP2881138 discloses an infusion set including a first flow channel constituted by a pliable tube and including a spike provided at one end thereof, a second flow channel constituted by a pliable tube and including a lock connector provided at one end thereof, the lock connector including a lock mechanism that is brought into engagement with a female connector, a third flow channel constituted by pliable tubes and including a male connector provided at one end thereof, and a three-way stopcock to which another end of the first flow channel, another end of the second flow channel and another end of the third flow channel are connected. The three-way stopcock includes a handle that can be switched between a first position that brings the first flow channel and the third flow channel into communication with each other and a second position that brings the second flow channel and the third flow channel into communication with each other.

US2013/274702 discloses a connector cap which can suppress leakage of fluid, and a transfusion line connection apparatus for use with the connector cap. The connector cap includes a container having a first opening and a second opening; a seal member disposed in the first opening so as to separate the inside and the outside of the container, the seal member including an insertion passage formed therethrough whereby an injection tube of a male connector can be inserted from the outside to the inside of the container; and a hydrophobic filter which is disposed in the second opening.

### SUMMARY OF INVENTION

### PROBLEM TO BE SOLVED BY INVENTION

A problem to be solved by the present invention is to provide a novel infusion set that makes it possible to, by means of a simpler procedure, easily, conveniently, and definitively implement the priming operations that are carried out prior to use of the infusion set, and in which the risk of occurrence of damage to equipment and contamination of the hospital room interior due to unintentional leakage of liquid from the infusion set while priming operations are being carried out or after priming operations have been carried out is drastically reduced.

Furthermore, another problem to be solved by the present invention is to provide a novel infusion set which, by making it possible for an infusion container to be pierced on a first attempt, permits priming operations to be carried out in definitive fashion separately at a respective first member making up a set of primary tubing and a respective second member making up a set of secondary tubing in an infusion set without contamination of areas thereabout, and which, by thereafter making it possible, at any desired timing, for said set of secondary tubing at which the infusion container has been pierced to be made integral with and inseparable from the set of main tubing of the infusion set, permits an infusion to be administered in stable fashion without occurrence of contamination or misidentification of infusion administration procedures.

Furthermore, another problem to be solved by the present invention is to provide a novel infusion set that makes it possible to prevent bodily pain to patients and/or leakage of liquid that might otherwise occur due to twisting and/or tension acting on the infusion set and/or intravenous drip needle(s) while lowering the risk of occurrence of accidents in which the IV stand from which the infusion set is suspended falls over.

In addition, a problem to be solved by the present invention is to, also in addition to the foregoing, provide a novel infusion set that makes it possible to prevent occurrence of situations in which confusion as to order of use of a plurality of spikes provided at an infusion set when installing infusion container(s) causes error in the order in which infusions are to be administered, or in which infusion(s) containing different drug(s) become mixed up among multiple pieces of infusion tubing when there has been a change in set(s) of infusion(s) at the infusion set, and other accidents such as may occur due to human error, and which facilitates division of labor among workers through adoption of a constitution in which setup of a first member, and a second member making up a set of secondary tubing, can be carried out separately, and that moreover makes it possible to easily establish standard procedures for use.

### MEANS FOR SOLVING PROBLEM

A first means in accordance with the present invention for solving the foregoing problems is an infusion set as defined in claim 1.

A preferred second means in accordance with the present invention for solving the foregoing problems is the infusion set according to claim 1 further limited by the features of claim 2.

A preferred third means in accordance with the present invention for solving the foregoing problems is the infusion set according to claim 2 further limited by the features of claim 3.

A preferred fourth means in accordance with the present invention for solving the foregoing problems is the infusion set according to claim 3 further limited by the features of claim 4.

### BENEFIT OF THE INVENTION

Because an infusion set in accordance with the present invention is provided in the form of an infusion set which is ready for use, there is no need for a pharmacist, physician, or nurse to assemble same in correspondence to each patient who is to be the target of medical treatment. In addition, it is possible to obtain the benefit whereby priming and backpriming are completed in extremely rapid and simple fashion by causing air within the infusion set to be quickly discharged therefrom not only at primary tubing branch(es) but also at secondary tubing branch(es), and simultaneously causing same to be filled with physiological saline solution, as a result of only a single operation in which a spike at an upstream-most location in said infusion set is inserted in an infusion container having sealed therewithin physiological saline solution which is suspended from an IV stand.

Furthermore, no physiological saline solution whatsoever is discharged from cap(s) which is/are installed at spike(s) and connector(s) for connection with intravenous drip needle(s) and at which hydrophobic filter(s) is/are arranged, and so damage to equipment and contamination do not occur. By causing said cap(s) to be provided with clip(s) equipped with catch(es) for flange(s) arranged at spike(s), the risk that cap(s) will become disengaged during priming and backpriming operations is lowered. Because lid(s) provided on cap(s) at which hydrophobic filter(s) is/are arranged are closed following priming and backpriming, physiological saline solution at regions peripheral to hydrophobic filter(s) at cap interior(s) does not spill out from cap insertion port(s). In addition, because the amount of physiological saline solution that is used can be kept to a minimum, such that an adequate remaining amount is retained therewithin, this permits effective use thereof, as there may be no need to replace the infusion container at the time of any medical treatment that may take place thereafter.

Furthermore, in an infusion set in accordance with the present invention, causing instant tubing of a plurality of three-way stopcocks arranged at various locations to be arranged to the left and the right in balanced fashion will make it possible to achieve a situation in which no abnormal force acts on infusion tubing and/or intravenous drip needle(s), and in which the risk of occurrence of accidents in which intravenous drip needle(s) become detached or there is leakage of liquid is greatly reduced.

In addition, at the infusion set of the present invention, three-way stopcock(s) at which infusion tubing is connected to secondary tubing branch connector(s), said infusion tubing, shutoff clamp(s) linked thereto, and cap(s) are grouped together as a single group such that one or more thereamong is colored with the same color. In particular, where there are a plurality of said three-way stopcocks, groups belonging to adjacent three-way stopcocks are colored so as to be respectively different colors. This makes it possible to obtain the benefit whereby an order of colors is associated with an order of administration of infusions and/or a procedure for using the infusion set, making it possible to facilitate identification of sets of secondary tubing of different groups.

Moreover, at the infusion set of the present invention, by causing the aforesaid colors and the order thereof to be adopted from those in a familiar verse or lyric of a nursery rhyme, and by moreover causing illustration(s) in which colored images associated with the aforesaid verse or lyric of a nursery rhyme based on which these were adopted to be displayed in layered fashion on a package within which the infusion set is sealed, it will be possible to naturally and accurately recognize the order of colors, i.e., the procedure for use of the infusion set and/or the order of administration of infusions, making easy memorization in association with such familiar content possible, and making it possible to more effectively prevent misidentification of administration procedures. In addition, when this is viewed by patients, pharmacists, nurses, and physicians, it will alleviate tension, and will make it possible to simultaneously obtain a psychological effect whereby the atmosphere at sites at which medical treatment is carried out is made more relaxing.

In addition, provision of the infusion set of the present invention will permit smooth progress to be made in standardization of operational procedures for use of the infusion set, and will make it possible to obtain the benefit whereby medical accidents such as mistaken administration or the like occurring as a result of misidentification by a pharmacist, physician, or nurse, or other such human error, are prevented.

### BRIEF DESCRIPTION OF THE DRAWINGS

[FIG. 1] Explanatory diagram showing an infusion set (in which secondary tubing branch connectors at three-way splitters are needle-less ports) in accordance with the present invention. (a) indicates first member; (b) indicates second members.
[FIG. 2] Explanatory diagram showing an infusion set in accordance with the present invention.
[FIG. 3] Explanatory diagram showing an infusion set in accordance with the present invention.
[FIG. 4] Explanatory diagram showing an infusion set in accordance with the present invention.
[FIG. 5] Drawing for explaining a method for carrying out priming in the context of an infusion set in accordance with the present invention.
[FIG. 6] Drawing for explaining a method for carrying out priming in the context of an infusion set in accordance with the present invention.

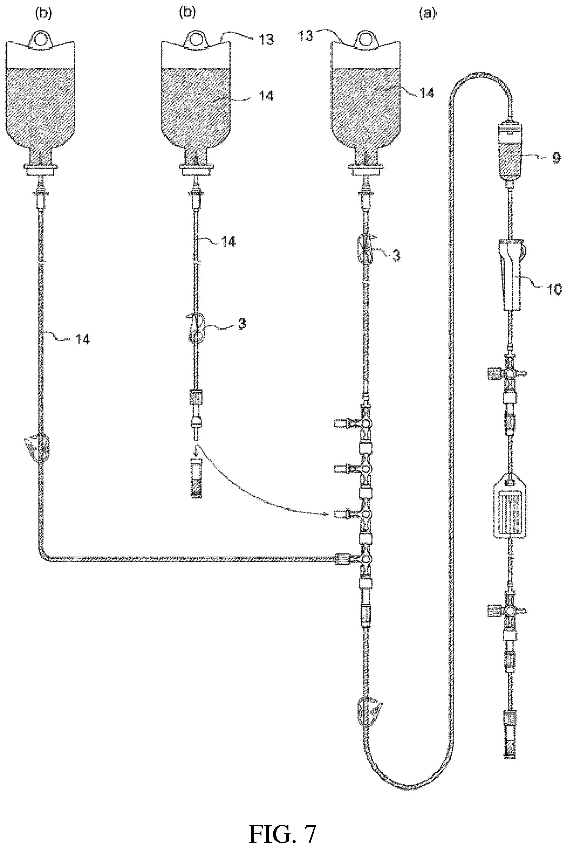

[FIG. 7] Drawing for explaining a method for carrying out priming in the context of an infusion set in accordance with the present invention.

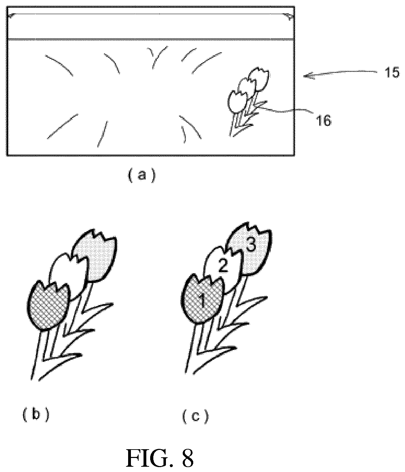

[FIG. 8] Drawing for explaining an infusion set in accordance with the present invention that is sealed within a package at which displayed in mutually offset and partially overlapping fashion on the surface thereof are a plurality of illustrations 16. (a) is an explanatory diagram showing the external appearance of a package. (b) is a drawing for explaining the coloration that is applied to the illustrations. (c) is a drawing for explaining numbers indicating order of administration that are further displayed on the illustrations.
[FIG. 9] Explanatory diagram showing an infusion set (in which secondary tubing branch connectors at three-way splitters are needle-less ports and in which connectors for respective sets of instant tubing are arranged so as to be distributed to left and to right) in accordance with the present invention.

### EMBODIMENTS FOR CARRYING OUT THE INVENTION

Below, embodiments for carrying out the present invention are described as appropriate with reference to the drawings.

### Configuration and Constitution of Infusion Set of Present Invention

An infusion set in accordance with the present invention comprises a first member constituting primary tubing in the infusion set, and second member(s) constituting secondary tubing in the infusion set.

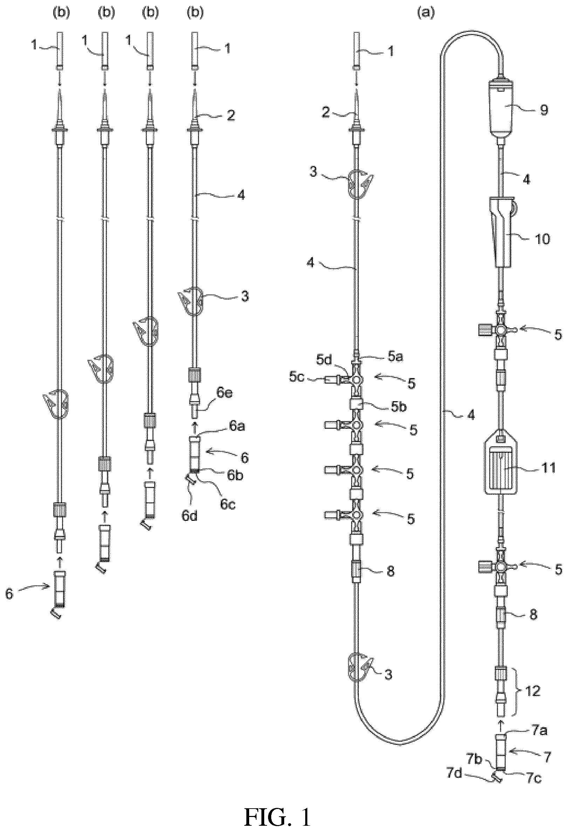

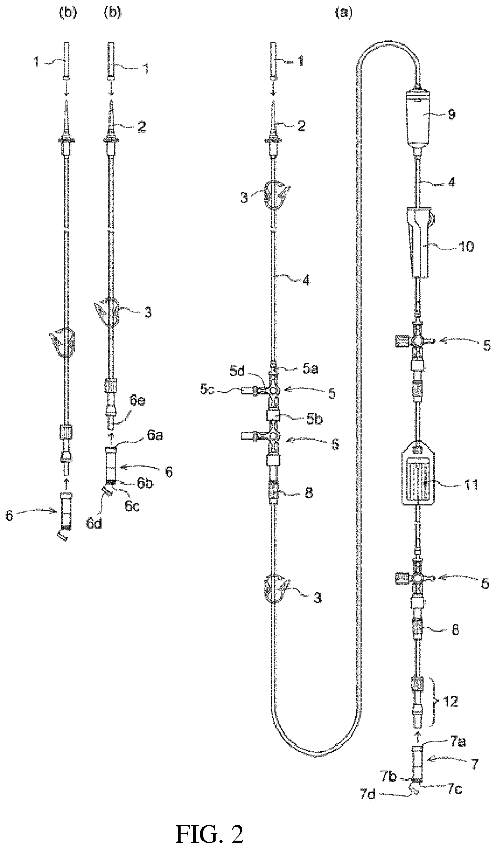

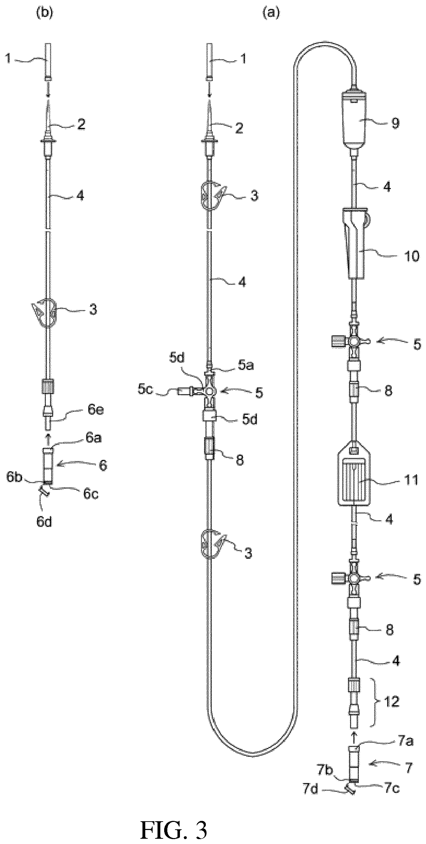

The first member constituting primary tubing in the infusion set has spike(s) 2; optional spike cap(s) 1; three-way stopcock(s) 5; male connector(s) 8; drip chamber(s) 9; roller clamp(s) 10; connector(s) 12 for connection with intravenous drip needle(s); cap(s) 7 for installation on connector(s) for connection with intravenous drip needle(s), arranged at which there is/are opening(s) not allowing passage therethrough of solid(s) or liquid(s) but allowing passage therethrough of gas(es), hydrophobic filter(s) being arranged at location(s) inward from where tip(s) of connector(s) for connection with intravenous drip needle(s) is/are inserted at interior(s) of cap(s) by way of insertion port(s) for connector(s) for connection with intravenous drip needle(s), lid(s) for closing said opening(s) being arranged at exterior(s) of said opening(s); infusion tubing 4; shutoff clamp(s) 3 for pressing on and opening and/or closing flow path(s) within infusion tubing; and optional filter bag(s) 11 ((a) at FIGS. 1 through 4).

Furthermore, the second member(s) constituting secondary tubing in the infusion set have spike(s) 2; optional spike cap(s) 1; infusion tubing 4; shutoff clamp(s) 3 for pressing on and opening and/or closing flow path(s) within infusion tubing; connector(s) 6e for connection with three-way stopcock(s); and cap(s) 6 for installation on connector(s) for connection with three-way stopcock(s), arranged at which there is/are opening(s) not allowing passage therethrough of solid(s) or liquid(s) but allowing passage therethrough of gas(es), hydrophobic filter(s) being arranged at location(s) inward from where tip(s) of connector(s) for connection with three-way stopcock(s) is/are inserted at interior(s) of cap(s) by way of insertion port(s) for connector(s) connected to said three-way stopcock(s), lid(s) for closing said opening(s) being arranged at exterior(s) of said opening(s) ((b) at FIGS. 1 through 4).

In addition, the infusion set is such that, prior to use thereof, the first member constituting primary tubing and the second member(s) constituting secondary tubing in the infusion set are in a mutually separate state. For this reason, it is possible for the first member constituting primary tubing and the second member(s) constituting secondary tubing in the infusion set to remain in a mutually separate state as the interiors thereof are filled with physiological saline solution or other such solution for the purpose of causing these to be made to assume a primed state. In addition, during use, when infusion container(s) are connected to the infusion set, secondary tubing branch connector(s) for three-way stopcock(s) at the first member and connector(s) connected to three-way stopcock(s) at the second member(s) are connected, causing the first member constituting primary tubing and the second member(s) constituting secondary tubing in the infusion set to be in the form of a single integral unit. So as to prevent leakage of solution during use as an infusion set, it is preferred that connections therebetween be constituted in such fashion as to make it impossible for these to again become separate.

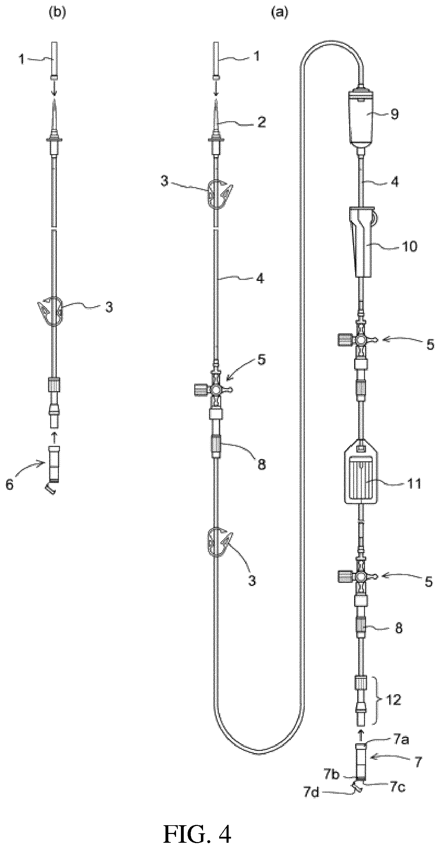

In addition, the respective members are connected so as to have constitution(s) as indicated below. That is, the first member constituting primary tubing in an infusion set in accordance with the present invention is equipped with spike(s) 2 at upstream-most location(s) thereat, said spike(s) being optionally covered with spike cap(s), said spike(s) being connected to one end of infusion tubing 4 equipped with shutoff clamp(s) 3 for pressing on and opening and/or closing flow path(s) within infusion tubing, the other end of said infusion tubing 4 being connected to primary tubing upstream branch connector(s) 5a of three-way stopcock(s) 5. In addition, connected to primary tubing downstream branch connector(s) of said three-way stopcock(s) there is/are optionally one or more three-way stopcock(s) 5. In addition, male connector(s) 8 is/are connected to primary tubing downstream branch connector(s) 5b of the aforesaid three-way stopcock(s) 5 disposed at downstream location(s), one end of infusion tubing 4 equipped with shutoff clamp(s) 3 for pressing on and opening and/or closing flow path(s) within infusion tubing being further connected thereto, drip chamber(s) 9 being connected to the other end of said infusion tubing 4. In addition, said drip chamber(s) 9 is/are connected to one end of infusion tubing 4 on which roller clamp(s) 10 is/are installed, the other end of said infusion tubing 4 being connected to primary tubing upstream branch connector(s) 5a of three-way stopcock(s) 5. In addition, primary tubing downstream branch connector(s) 5b of said three-way stopcock(s) 5 is/are connected to male connector(s) 8, one end of infusion tubing 4 being further connected thereto, and optionally filter bag(s) 11 and infusion tubing 4 downstream therefrom being connected to the other end of said infusion tubing 4. Moreover, the other end of said infusion tubing 4 is connected to primary tubing upstream branch connector(s) 5a of three-way stopcock(s) 5. In addition, the constitution of this is in a state such that primary tubing downstream branch connector(s) 5b of said three-way stopcock(s) 5 is/are connected to male connector(s) 8, one end of infusion tubing 4 being further connected thereto, and the other end of said infusion tubing 4 being connected to connector(s) 12 for connection with intravenous drip needle(s) covered with cap(s) 7 for installation on connector(s) for connection with intravenous drip needle(s), arranged at which there is/are opening(s) not permitting passage therethrough of solid(s) or liquid(s) but permitting passage therethrough of gas(es), hydrophobic filter(s) being arranged at location(s) inward from where tip(s) of connector(s) for connection with intravenous drip needle(s) is/are inserted at interior(s) of cap(s) by way of insertion port(s) for connector(s) for connection with intravenous drip needle(s), and lid(s) for closing said opening(s) at exterior(s) of said opening(s). As the foregoing three-way stopcock(s), note that three-way stopcock(s) at which secondary tubing branch connector(s) is/are needle-less port(s) may be employed, and/or those having general-purpose three-way stopcock(s) may be employed (FIG. 4). Because the first member and the second member are linked following priming, it is preferred that three-way stopcock(s) at which secondary tubing branch connector(s) is/are needle-less port(s) which facilitate procedures such as those in which linked location(s) is/are wiped off and cleaned be employed Where general-purpose three-way stopcock(s) are employed, it is preferred that these be in a state such that they are covered with cap(s) until such time as the second member(s) are connected to secondary tubing branch connector(s).

Furthermore, the second member(s) constituting secondary tubing in the infusion set in accordance with the present invention are equipped with spike(s) 2 at upstream-most location(s) therein. In addition, said spike(s) are optionally covered with spike cap(s) 1. Moreover, said spike(s) are connected to one end of infusion tubing 4 equipped with shutoff clamp(s) 3 for pressing on and opening and/or closing flow path(s) within infusion tubing. In addition, the other end of said infusion tubing 4 is connected to connector(s) 6e for connection with three-way stopcock(s). The constitution of this is in a state such that said connector(s) for connection with three-way stopcock(s) are covered with cap(s) 6 for installation on connector(s) for connection with three-way stopcock(s), arranged at which there is/are opening(s) not permitting passage therethrough of solid(s) or liquid(s) but permitting passage therethrough of gas(es), hydrophobic filter(s) being arranged at location(s) inward from where tip(s) of connector(s) for connection with three-way stopcock(s) is/are inserted at interior(s) of cap(s) by way of insertion port(s) for said connector(s) for connection with three-way stopcock(s), and lid(s) for closing said opening(s) at exterior(s) of said opening(s).

These first member and second member(s) are such that the respective members are connected in advance so that the first member and the second member(s) are each respectively in the form of a single integral unit, these being provided to a user as an infusion set capable of instant use when the first member and the second member(s) are primed and these are connected.

Where it is necessary to remove debris or the like such as may be produced when spike(s) 2 pierce infusion container(s) 13 and/or other such solids such as may be present within infusion(s), the aforesaid filter bag(s) 11 is/are incorporated in the infusion set, and the respective members are connected in advance in the form of single integral unit(s) provided as an infusion set capable of instant use. In situations where a formulation contraindicated for use with filter bags due to the possibility that it may cause clogging is employed, in situations where no solids are present within the infusion, and in situations where a formulation having high viscosity for which it would be difficult to ensure achievement of adequate drip rate of the infusion at the time of intravenous drip infusion is employed, a constitution is employed in which said aforesaid filter bag(s) 11 is/are omitted, and the respective members are connected in advance in the form of single integral unit(s) provided as an infusion set capable of instant use.

In accordance with the infusion set of the present invention, three-way stopcock(s) 5 at which infusion tubing 4 is connected to secondary tubing branch connector(s) 5c, said infusion tubing 4, shutoff clamp(s) 3 linked thereto, spike(s) 2, and cap(s) 1, 6, 7 may be grouped together as a single group such that one or more thereamong is made to have location(s) of the same color. Moreover, where said infusion set has a plurality of three-way stopcocks 5 at which spikes 2 are connected to secondary tubing, groups belonging to adjacent three-way stopcocks 5 may be made to have location(s) of respectively different colors. Where coloring is carried out, respective groups are colored so as to be of different colors in correspondence to the order in which infusion(s) is/are to be administered so as to permit identification thereof.

However, despite attempts to memorize whatever unremarkable order of colors may have been established, where there is no deep meaning attached to the colors and/or order thereof, there is a risk that the colors and/or order thereof will be misidentified, for which reason particularly careful attention is demanded of nurses and physicians.

To avoid risk of misidentification, it is preferred that colors and an order thereof such as appear in a nursery rhyme, lyric, or verse that many people will know, having been familiar therewith from a young age, be employed as the colors and order thereof for identifying the aforesaid respective groups. For example, where provided in Japan, the colors and order indicated by "red, white, yellow" appearing in lyrics about tulips in the nursery rhyme might be employed; or where provided in an English-speaking region, the colors and order indicated by "red, blue, white" in the verse which goes roses are red, violets are blue, sugar is sweet, and so are you that comes from Mother Goose might be employed, respective groups being colored with different colors in order of use so as to permit identification thereof.

By thus causing an infusion set to employ coloration constituted so as to permit colors and order thereof to be memorized in association with lyrics, verses, and/or other such familiar content, as compared with memorization of an unremarkable order of colors established in the context of standardized administration procedures, nurses and physicians can more naturally and accurately recognize order of colors, i.e., procedures for use of the infusion set and/or order of administration of infusions, and as easy memorization in association with such familiar content is facilitated, it is possible to more effectively prevent misidentification of administration procedures. Moreover, this will permit smooth progress to be made in standardization of operational procedures for preventing misidentification of administration procedures at sites where many pharmacists, nurses, and physicians are engaged in medical treatment.

In addition to the aforesaid coloration, it is possible by further optionally causing any among three-way stopcock(s), infusion tubing, shutoff clamp(s), spike(s), cap(s), and/or colored tape(s) to be in a state in which number(s) indicating order(s) of administration are displayed thereon, to even more effectively prevent misidentification of standardized administration procedures.

An infusion set provided with coloration and optionally with display of numbers indicating order of administration as described above is sealed within a pouch or other such package 15 and is provided to a site at which medical treatment is carried out. Here, to even further increase the effect whereby misidentification of standardized administration procedures may be prevented as a result of coloration and display of number(s) indicating order(s) of administration, the infusion set may be sealed within a package at which displayed in mutually offset and partially overlapping fashion on the surface thereof are a plurality of illustrations ((a) at FIG. 11). It is preferred that the infusion set be such that the foregoing illustrations are respectively colored so as to have the same colors as the coloration given to groups within the infusion set comprising the aforesaid three-way stopcocks at which infusion tubing is connected to secondary tubing branch connectors, said infusion tubing, shutoff clamps linked thereto, spikes, and caps for installation on spikes, and so as be colored in order from the frontmost illustration to the backmost illustration in correspondence to the order of use ((b) at FIG. 11). It is more preferred that the aforesaid colored illustrations be such that numbers indicating order of administration be displayed thereon in ascending order from the frontmost illustration to the backmost illustration ((c) at FIG. 11).

From frontmost to backmost, the illustrations on the aforesaid package should respectively employ, and be respectively colored, in order of appearance of images associated with a familiar verse or lyric of the nursery rhyme based on which coloration of the infusion set is carried out. By thus providing an infusion set sealed within a package displaying illustrations, nurses and physicians can, merely by looking at the package before or after the package is opened, extremely naturally and without difficulty grasp the colors and the order thereof, i.e., the order of administration, memorized in association with lyrics, verses, and/or other such familiar content, making it possible to more effectively prevent misidentification of standardized administration procedures. Moreover, by causing numbers indicating order of administration to be displayed at the respective colored illustrations, the order of use of the respective sets of infusions in the infusion set can be linked to colors and definitively recognized, making it possible to greatly reduce the likelihood of occurrence of human error.

Moreover, an infusion set sealed within a package displaying illustrations such as images associated with the aforesaid familiar verse or lyric of a nursery rhyme, when viewed by patients, nurses, and physicians will also alleviate tension, making it possible to simultaneously obtain a psychological effect whereby the atmosphere at sites at which medical treatment is carried out is made more relaxing.

### Materials

There is no particular limitation with regard to the materials that may be employed for formation of the members that make up the infusion set of the present invention, it being possible to use materials such as are ordinarily employed in the context of members for infusion sets and medical equipment; for example, Nylon, polycarbonate, polypropylene, polystyrene, and/or other such resin materials and/or stainless steel and/or other such metals may be employed, it being possible to employ polyolefinic resins and/or other such materials suitable for soft tubing at the infusion tubing. Depending on the type of drug, e.g., anticancer agent, used, it may be the case that plasticizer will leach out therefrom. Resin material(s) employed for formation of the members that make up the infusion set of the present invention therefore employ substance(s) free of polyvinyl chloride (PVC) and/or PVC that does not contain DEHP (di-2-ethylhexyl phthalate), or else Nylon and/or polycarbonate is employed. Hydrophobic filter(s) may employ polytetrafluoroethylene (PTFE), polyethylene (PE), polyolefin, polypropylene, polyethylene, polyvinylidene fluoride (PVDF), nitrocellulose, and/or the like, it being preferred that polyethylene (PE) and/or polytetrafluoroethylene (PTFE) be employed therefor. Any of the various foregoing resin materials may be employed in colored form. Furthermore, stainless steel and/or other metals may be employed in a form in which the surface thereof has undergone coloration treatment, in which case it is preferred that material(s) which have undergone coloration treatment that is highly anticorrosive be employed.

### Manufacturing Operations

The infusion set of the present invention is manufactured in such fashion that the respective members making up the first member and the second member(s) are definitively connected so as to form an infusion set in the form of single integral unit(s). There is no particular limitation with regard to the method for definitively connecting the respective members, it being possible to use methods such as are ordinarily employed as methods for obtaining an infusion set or medical equipment in the form of single integral unit(s); for example, adhesive operations employing adhesives ordinarily used for infusion sets or other such medical equipment, fusing operations that make use of heat, ultrasonic waves, or the like, and/or other such techniques may be utilized. By providing the infusion set in the form of single integral unit(s), the risk that joined parts will become separated is eliminated, making it possible to more definitively prevent medical accidents such as damage to equipment and/or contamination of the hospital room interior due to occurrence of unintentional leakage of liquid.

Moreover, so as to permit immediate commencement of priming operations after the package has been opened and the infusion set has been removed therefrom, an infusion set in accordance with the present invention may be provided in presterilized form. There is no particular limitation with regard to the method for sterilization of the infusion set, it being possible to use methods such as are ordinarily employed as methods for sterilization of infusion sets and/or medical equipment; for example, methods which include ethylene oxide gas sterilization, γ irradiation sterilization, e-beam sterilization, radiation sterilization, ultraviolet irradiation sterilization, hydrogen peroxide sterilization, and ethanol sterilization may be employed. In addition, as said sterilization method, it is preferred based on considerations which include ease of manufacturing and cost reduction that ethylene oxide gas sterilization, e-beam sterilization, and/or γ irradiation sterilization be employed. It is preferred that e-beam sterilization be carried out to such a degree as will not cause degradation of the infusion set, and it is preferred that the irradiative energy during γ irradiation sterilization be within a range that is up to on the order of 5 kGy to 30 kGy so as to cause sterilization to be carried out to such a degree as will not cause degradation of the infusion set.

### Procedure for Using Infusion Set of Present Invention

At the infusion set of the present invention, priming operations are first respectively carried out at the first member and the second member(s) so as to remove air from the interior of the infusion set and cause it to be filled with physiological saline solution or other such solution.

Package 15 within which the infusion set is sealed is opened, and investigation is carried out to make sure that, at the first member and at the second member(s) for instant use in the infusion set, cap(s) 6 for installation on connector(s) for connection with three-way stopcock(s) provided with lid(s) and cap(s) 7 for installation on connector(s) for connection with intravenous drip needle(s) provided with lid(s) are definitively installed thereon, and to make sure that lid(s) 6d, 7d of the respective caps are open. And at the second member(s) for instant use as well, investigation with respect to the state(s) of cap(s) is carried out in similar fashion. Furthermore, investigation is carried out to make sure that all shutoff clamp(s) 3 are in their open state(s).

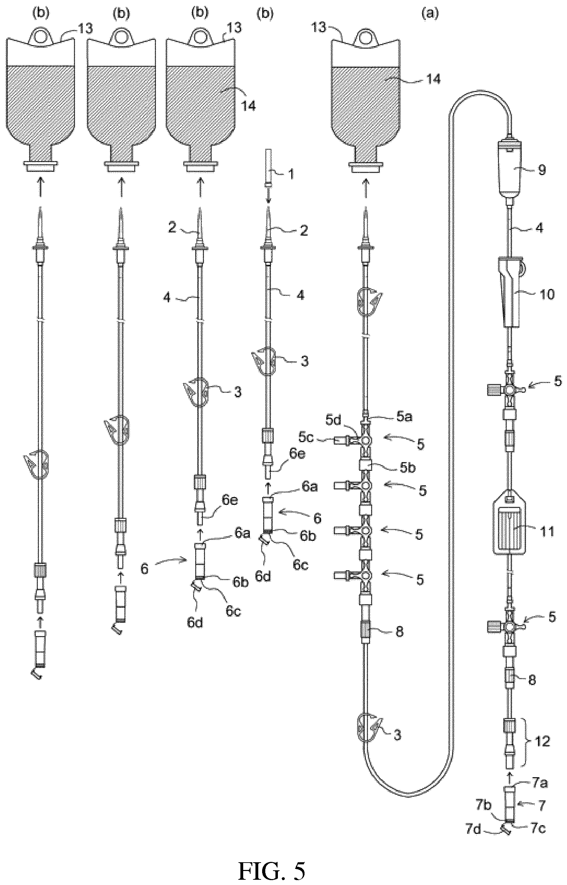

Where three-way stopcock(s) constituting link(s) to the second member(s) have lever(s) 5d, investigation is carried out to make sure that these are positioned so as to permit flow to/from all three branches. Furthermore, where three-way stopcock(s) at which there is no spike or infusion tubing connected at secondary tubing branch(es) for use as emergency port(s) have lever(s) 5d, investigation is carried out to make sure that these are in position(s) permitting flow to/from primary tubing upstream branch(es) and primary tubing downstream branch(es) but not permitting flow to/from secondary tubing branch(es). Furthermore, investigation is carried out to make sure that roller clamp(s) 3 are in their open state(s) (FIG. 5).

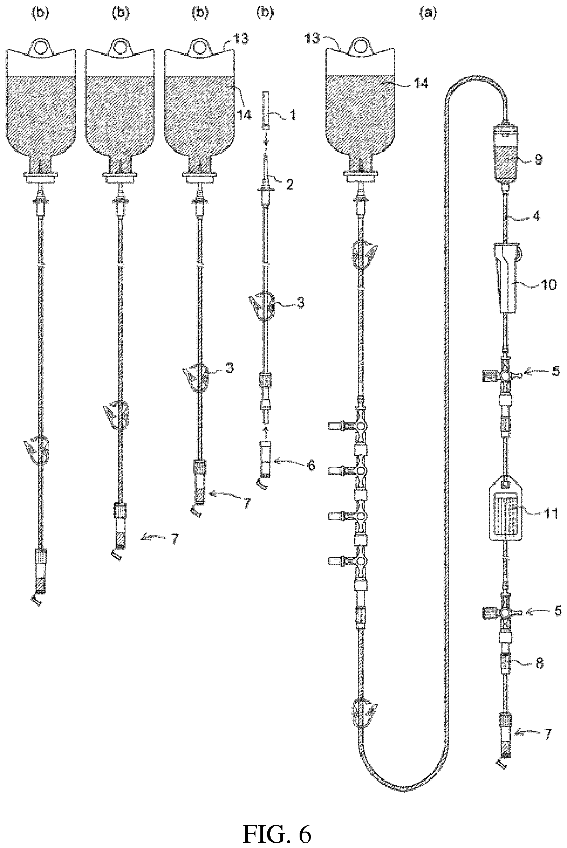

Next, infusion containers 13 having sealed therewithin physiological saline solution 14 for use during priming of the first member and the second member(s) in an infusion set in accordance with the present invention are respectively suspended from IV stand(s) or other such stand(s). Next, cap(s) 1 for installation on spike(s) installed on spike(s) disposed at upstream-most location(s) at the first member and the second member(s) in an infusion set in accordance with the present invention is/are removed. In addition, spike 2 of the first member is used to pierce the infusion container 13 for priming the first member, spike 2 of the second member is used to pierce the infusion container 13 for priming the second member, and respective priming operations are initiated (FIG. 5 and FIG. 6). At the first member, in the event that secondary tubing branch connector(s) employ three-way stopcock(s) having needle-less port(s), such three-way stopcock(s) are secured by means of securing hardware with which the stand(s) are equipped. Furthermore, where IV stand(s) are equipped with stay rod(s) for securing drip chamber(s) 9 or other such securing hardware, these may be used to further secure the foregoing. Note that it is preferred even where securing is not carried out during priming that securing hardware be used to secure three-way stopcock(s) 5 and drip chamber(s) 9 when initiating intravenous drip infusion, to prevent situations in which the entire weight of the infusion set is borne by intravenous drip needle(s) and connector(s) 12 for connection with intravenous drip needle(s), so that region(s) at which intravenous drip needle(s) are inserted are not placed under stress.

Physiological saline solution 14 flows into infusion tubing 4 from infusion container(s) 13 placed on spike(s) 2 disposed at upstream-most location(s), and priming operations commence. As physiological saline solution 14 displaces air within infusion tubing 14 of the first member, it flows from primary tubing upstream branch connector 5a of the three-way stopcock 5 which is disposed downstream therefrom into three-way stopcock 5. Because lever 5d of said three-way stopcock 5 is positioned so as to permit flow to/from all three branches, as physiological saline solution 14 flowing thereinto displaces air within three-way stopcock 5, it further flows into primary tubing downstream branch connector 5b, and further causes air within infusion tubing 4 which is connected thereto to be displaced toward a location downstream therefrom. In addition, as physiological saline solution 14 which has flowed thereinto displaces air from within infusion tubing 4 further connected thereto to a location downstream therefrom, it flows into the interior of three-way stopcock 5 and filter bag 11 which are connected at the far end thereof, and as it displaces air from within infusion tubing 4 further connected thereto to a location downstream therefrom, it flows therealong to flow into connector 12 for connection with an intravenous drip needle.

After flowing into connector 12 for connection with an intravenous drip needle, physiological saline solution 14, as it displaces the air therewithin to a location downstream therefrom, flows from the tip of connector 12 for connection with an intravenous drip needle into the interior of cap 7 which is for installation on a connector for connection with an intravenous drip needle, arranged at which there is an opening not permitting passage therethrough of liquid(s) or solid(s) but permitting passage therethrough of gas(es) at the interior of the cap, a hydrophobic filter being arranged at a location inward from where the tip of the connector for connection with an intravenous drip needle is inserted at the interior of the cap by way of the insertion port for the connector for connection with an intravenous drip needle, and a lid for closing said opening at the exterior of said opening. In addition, physiological saline solution 14 which has flowed thereinto causes air that had been present in the region peripheral thereto to further pass through hydrophobic filter 7b and to be displaced to the exterior by way of cap opening 7c, as a result of which the region peripheral thereto becomes filled with physiological saline solution 14. Here, hydrophobic filter 7b does not permit passage therethrough of physiological saline solution 14. Furthermore, air remains in the portion toward the base from the tip of connector 12 for connection with an intravenous drip needle from which physiological saline solution 14 flows at the interior of cap 7.

In similar fashion as at the first member, at the second member as well, physiological saline solution 14 flows into infusion tubing 4 from infusion container 13 placed on spike 2 disposed at the upstream-most location, and priming operations commence. As physiological saline solution 14 displaces air within infusion tubing 14 of the second member, it displaces air from within infusion tubing 4 connected thereto to a location downstream therefrom, and as it does so it flows therealong to flow into connector 6e for connection with a three-way stopcock. In addition, physiological saline solution 14 which has flowed thereinto causes air that had been present in the region peripheral thereto to further pass through hydrophobic filter 6b and to be displaced to the exterior by way of cap opening 6c, as a result of which the region peripheral thereto becomes filled with physiological saline solution 14. Here, hydrophobic filter 6b does not permit passage therethrough of physiological saline solution 14. Furthermore, within cap 6, air remains in the portion that is more toward the base than the tip of connector 12 for connection with a three-way stopcock from which physiological saline solution 14 flows.

In this way, at an infusion set in accordance with the present invention, priming is completed by causing air within each the first member and the second member of said infusion set to be quickly discharged therefrom and simultaneously causing said infusion set to be filled with physiological saline solution as a result of only a single operation in which "spike(s) 2 disposed at upstream-most location(s) in said infusion set are inserted in infusion container(s) 13 which have sealed therewithin physiological saline solution 14 for use during priming and which are suspended from IV stand(s)" (FIG. 6). In addition, regardless of which of the foregoing situations applies, because hydrophobic filter(s) 6b, 7b is/are present at cap(s) 6 for installation on connector(s) for connection with three-way stopcock(s) and at cap(s) 7 installed on connector(s) for connection with intravenous drip needle(s), there will be no occurrence of leakage of liquid even if lid(s) is/are open.

During the aforesaid priming operations, because hydrophobic filter(s) 6b, 7b is/are present, there will be absolutely no discharge of unwanted liquid, and there will be no occurrence of damage to equipment and/or contamination of the hospital room interior. Moreover, because the amount of physiological saline solution sealed within infusion container(s) connected to spike(s) at upstream-most location(s) that is used can be kept to a minimum, such that an adequate remaining amount is retained therewithin, this permits effective use thereof, as there may be no need to replace the infusion container(s) at the time of medical treatment that may take place thereafter. Furthermore, because an infusion set in accordance with the present invention is assembled in advance in the form of single integral unit(s), there being no occurrence of unintentional leakage of liquid during priming and backpriming operations, there will be no occurrence of damage to equipment and/or contamination of the hospital room interior.

After completion of priming and backpriming, lid(s) 6d, 7d at cap(s) for installation on connector(s) for connection with three-way stopcock(s) and at cap(s) for installation on connector(s) for connection with intravenous drip needle(s) are closed. In addition, shutoff clamp(s) 3 disposed at respective set(s) of infusion tubing 4 is/are pinched together, and roller clamp(s) 10 at the first member is/are moreover moved to their closed position(s), to close off the respective set(s) of infusion tubing 4. Furthermore, lever(s) 5d at three-way stopcock(s) 5 is/are moved to position(s) corresponding to the intravenous drip infusion(s) to be initiated. Investigation is carried out to make sure that there is no leakage of physiological saline solution 14.

Next, the interior of an infusion container connected to the second member is filled with anticancer agent and this is agitated in homogeneous fashion to prepare a therapeutic solution.

When preparatory procedures at the first member and the second member which will be used have been completed, those members are moved to the patient's bedside. At the patient's bedside, connector 6e for connection with three-way stopcock at the second member is connected and secured to secondary tubing branch connector 5c at a three-way stopcock of the first member. Where items divided into groups by color and/or number are used at the respective members, connection is carried out after first investigating to make sure that the secondary tubing branch connector 5c at the three-way stopcock and the connector 6e for connection with the three-way stopcock are of matching color and/or number group. This will make it possible to avoid mistaken connections that might otherwise occur due to human error. Note that where secondary tubing branch connector 5c at the three-way stopcock and connector 6e for connection with the three-way stopcock are equipped with securing fixtures for securing these, investigation is carried out to make sure that these are definitively secured. Prior to connection, note that connector 6e for connection with three-way stopcock is wiped off or other such procedures are carried out to cause it to be cleaned as appropriate in advance.

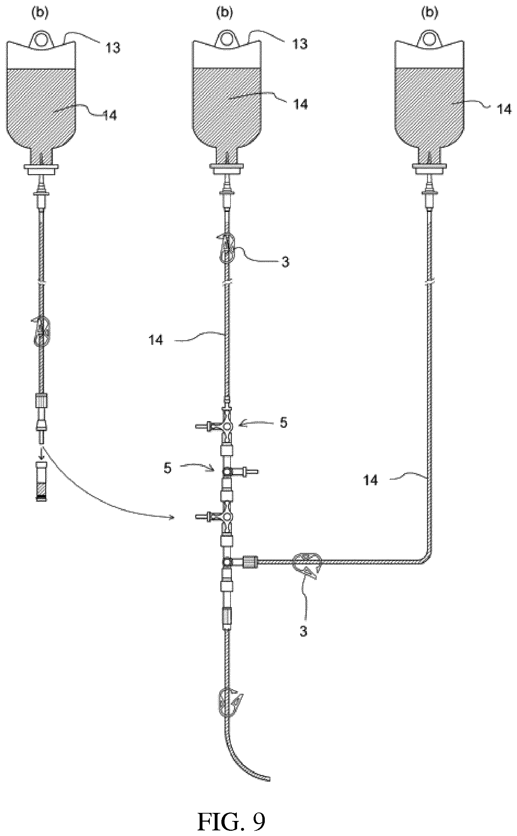

Where a plurality of infusion sets are used, there is a need to prevent accidents in which intravenous drip needle(s) become detached or there is leakage of liquid from region(s) at which intravenous drip needle(s) are inserted due to application of torsional forces on infusion tubing or accidents in which IV stand(s) fall over due to changes in the distribution of weight. Furthermore, where an anticancer agent or the like is administered by intravenous drip infusion, because particularly long times are required, there is a need to accommodate the fact that the patient will move about and to implement measures to prevent accidents of the same type as described above. Where the present infusion set is such that a plurality of second members are connected, it is therefore preferred that orientations of secondary tubing branch connectors at the plurality of three-way stopcocks disposed therein be such that those which are employed are distributed to both the effect and the right or that some other such strategy be utilized to cause the balance in the distribution of weight at the infusion set to be maintained (FIG. 9). By so doing, the risk that an abnormal force will act on the intravenous drip needle, and the risk of occurrence of accidents in which the intravenous drip needle becomes detached or there is leakage of liquid, will be greatly reduced.

Where an infusion set in accordance with the present invention is used to administer an intravenous drip infusion, the infusion may be allowed to drip under the influence of gravity with drip rate being adjusted by drip chamber 9 and roller clamp 10, or drip chamber 9 may be further combined with an infusion monitor for adjustment of drip rate. In addition, drip chamber 9 and/or the infusion monitor are secured to a securing stay rod or other such securing hardware with which the IV stand is equipped so as to prevent the entire weight of the infusion set from being borne by intravenous drip needle(s) and connector(s) 12 for connection with intravenous drip needle(s). Furthermore, an infusion pump may be further combined therewith for adjustment of drip rate. This is useful at times when flow rate is low due to use of filter bag(s) 11 or use of therapeutic infusion(s) having high viscosity. Even where heavy infusion monitor(s) and infusion pump(s) are used in combination, by freely rotating the region(s) below a plurality of three-way stopcocks 5 having primary tubing downstream branch connectors 5b equipped with mechanisms that connect in such fashion as to permit rotation about primary tubing as axis or axes which are arranged at various locations in an infusion set in accordance with the present invention, adjustment of balance and elimination of torsion at tubing can be easily carried out. As a result, no abnormal force will act on the intravenous drip needle, and the risk of occurrence of accidents in which the intravenous drip needle becomes detached or there is leakage of liquid will be greatly reduced.

Furthermore, at the first member, with regard to the possibility that there may be a plurality of sets of secondary tubing due to presence of a plurality of linked three-way stopcocks 5, it is necessary that adequate attention be paid so as not to confuse the order of use of secondary tubing. In particular, where an anticancer agent or other such drug contraindicated for mixture with different drug(s) is being used, the secondary tubing set connected to the downstream three-way stopcock is used first, and the secondary tubing set(s) connected to three-way stopcock(s) upstream therefrom is/are used thereafter in sequence.

### WORKING EXAMPLES

Indicated below are working examples of manufacture and use of infusion sets in accordance with the present invention. The present invention is not to be limited in any way by these descriptions.

Members for constructing an infusion set in accordance with the present invention were prepared in the form of a cap for installation on a spike; a spike; a three-way stopcock; a male connector; a drip chamber; a roller clamp; a connector for connection with an intravenous drip needle; infusion tubing; a shutoff clamp for pressing on and opening and/or closing flow path(s) within infusion tubing; an optional filter bag; a cap, for installation on a connector for connection with an intravenous drip needle, arranged at which there was an opening configured so as to not allow passage therethrough of solid or liquid but so as to allow passage therethrough of gas at the interior of the cap, a hydrophobic filter being arranged at a location inward from where the tip of the connector for connection with the intravenous drip needle was inserted at the interior of the cap by way of an insertion port for a connector for connection with an intravenous drip needle, and a lid at the exterior of said opening for closing said opening; a connector for connection with a three-way stopcock; and a cap, for installation on a connector for connection with a three-way stopcock, arranged at which there was an opening not allowing passage therethrough of solid or liquid but allowing passage therethrough of gas, a hydrophobic filter being arranged at a location inward from where the tip of the connector for connection with the three-way stopcock was inserted at the interior of the cap by way of an insertion port for a connector that connects with said three-way stopcock, and a lid at the exterior of said opening for closing said opening.

The foregoing members were connected to form a first member and second member(s) in the form of single integral units as described at the section entitled Configuration and Constitution of Infusion Set of Present Invention and the section entitled Manufacturing Operations. In addition, caps for installation on connectors for connection with three-way stopcocks provided with lids and caps for installation on connectors for connection with intravenous drip needles provided with lids were definitively installed thereon, lids of the respective caps were opened, all shutoff clamps were placed in their open states, levers of three-way stopcocks by way of which the second member(s) were connected and three-way stopcocks at which there were no spikes, infusion tubing, or the like connected at secondary tubing branches for use as emergency ports were moved to positions permitting flow to/from primary tubing upstream branches and primary tubing downstream branches but not permitting flow to/from secondary tubing branches, and roller clamp 3 was placed in its open state. As shown in FIG. 1, an infusion set comprising a first member and four second members was manufactured as Working Example 1.

As Working Example 2, an infusion set which had three three-way stopcocks at a first member, tape respectively colored, in order from the bottom, red, white, and yellow being respectively attached thereto so as to cause these to be recognizable, and which furthermore had second members corresponding thereto, to which tape respectively colored, in order, red, white, and yellow was respectively attached so as to cause these to be recognizable, but which in other respects was identical to Working Example 1, was manufactured.

As shown in FIG. 11, at Working Example 3, a package was prepared at which displayed on the surface thereof were illustrations that were images of tulips, the frontmost flower being colored red, the flower behind and overlapped by that one being colored white, and the backmost flower further overlapped by that one being colored yellow, the first member and the second members of the infusion set of Working Example 2 being sterilized and sealed therewithin to produce an infusion set.

As Comparative Example 1, an infusion set in which, instead of the caps having lids and hydrophobic filters employed at Working Example 1, caps were employed that differed only with respect to the fact that they were not provided with lids for closing openings, but which in other respects was identical to Working Example 1, was manufactured.

Two infusion containers having sealed therewithin physiological saline solution were suspended from an IV stand, the spikes respectively disposed at the upstream-most locations at the first member and the second member of the infusion set were made to pierce the rubber seals of said infusion containers, and priming operations were initiated. Testing was respectively carried out using the respective infusion sets at the respective Working Examples and at the respective Comparative Examples.

As a result, regardless of which infusion set was used, it was observed that priming of the infusion set could be completed as a result of only a single operation in which a spike was made to pierce the seal of an infusion container, the interiors of the first member and the second member(s) of the infusion set being filled with physiological saline solution, with only the air at the interior of the infusion set that was displaced by said physiological saline solution being exhausted by way of hydrophobic filters respectively provided at caps for installation on connectors for connection with three-way stopcocks and caps for installation on connectors for connection with intravenous drip needles. There was no leakage of physiological saline solution from any of the connections between/among the various members.

Next, following completion of priming, the lids for closing the openings at the caps, at which hydrophobic filters were disposed, installed at the first member and the second member(s) of the foregoing respective infusion sets were closed. Note that there were no lids on the caps at Comparative Example 1. In addition, shutoff clamps disposed at the respective sets of infusion tubing were pinched together, and the roller clamp was moved to its closed position, to close off the respective sets of infusion tubing.

Caps, at which hydrophobic filters were disposed, installed at the respective Working Examples and Comparative Example were removed therefrom.

As a result, because the lids were closed at the caps in the respective Working Examples, air did not pass through the hydrophobic filters to reenter the cap interiors, and as a result of the action of surface tension due to the physiological saline solution and atmospheric pressure from the openings at the caps, physiological saline solution at regions peripheral to the hydrophobic filters at the interior did not spill out from the insertion ports at the caps. Furthermore, physiological saline solution did not drip out of the connectors for connection with intravenous drip needles or the connectors for connection with intravenous drip needles after they were detached.

However, at Comparative Example 1, which did not have lids, following removal of the caps from the connectors for connection with intravenous drip needles and the connectors for connection with intravenous drip needles, air did pass through the hydrophobic filters provided at those caps to reenter the cap interiors, and physiological saline solution that had remained at the cap interiors leaked out via the insertion ports for the connectors for connection with intravenous drip needles and the connectors for connection with intravenous drip needles at the caps and spilled out to the exterior.

Furthermore, in the infusion sets at the working examples of the invention under application, because secondary member(s) constituting instant tubing were not initially connected to the first member constituting primary tubing in the infusion set, there being no unwanted infusion tubing or the like hanging down about the periphery of the first member, it was possible to easily gain access to more than enough space in which to carry out procedures, and it was possible for preparatory procedures at the infusion set to proceed in satisfactory fashion. Furthermore, with regard to the second member(s), because these were connected from the bottom one-at-a-time in order of use, there was no inconvenience due to presence of unwanted instant tubing such as can occur when using a conventional infusion set, as it was the case for unconnected locations at secondary member(s) constituting instant tubing as well that it was similarly possible to easily gain access to more than enough space in which to carry out procedures, and it was found that it was possible to safely and definitively carry out preparatory procedures at the infusion set and also the intravenous drip procedures employing the infusion set that took place thereafter.

When connecting the first member and the second member(s), air present at locations at which connectors were mutually connected was assimilated by locations at which air which was present at the drip chamber.

At the infusion set of Working Example 2, because portions of the members were colored, it was possible with just a glance to recognize the respective sets of secondary tubing that were to be connected with the respective members and the spikes at the far ends thereof.

Furthermore, at the infusion set of Working Example 3, the illustrations depicted on the surface of the package immediately caught the eye, and it was possible to effortlessly recognize that red, white, and yellow coloring was employed in order from the frontmost depicted image to the images that were depicted therebehind. In addition, when the package was opened, it was possible with just a glance to recognize that portions of the members making up the sets of secondary tubing in the infusion set that was sealed therewithin were colored with the same colors.

### INDUSTRIAL UTILITY

In accordance with the means of the present invention, priming and backpriming operations can be carried out as a result of only a single operation in which a spike is made to pierce the seal of an infusion container containing physiological saline solution, and, there being no unintentional leakage of liquid while operations are being carried out or after operations have been carried out, it is possible to drastically reduce the risk of occurrence of damage to equipment and contamination of the hospital room interior, and the physiological saline solution can also be utilized in effective fashion during any medical treatment that may take place thereafter. Furthermore, it is possible to provide a novel infusion set that makes it possible to easily perform operations for alleviation of twisting and operations for adjustment of position of infusion tubing and/or infusion container(s) while maintaining the stability of the infusion set, and to prevent bodily pain to patients and/or leakage of liquid that might otherwise occur due to twisting and/or tension acting on the infusion set and/or intravenous drip needle(s) while lowering the risk of occurrence of accidents in which the IV stand from which the infusion set is suspended falls over. Moreover, it is possible to provide a novel infusion set that makes it possible to prevent occurrence of situations in which confusion as to order of use of a plurality of spikes provided at an infusion set when installing infusion container(s) causes error in the order in which infusions are to be administered, or in which infusion(s) containing different drug(s) become mixed up among multiple pieces of infusion tubing when there has been a change in set(s) of infusion(s) at the infusion set, and other accidents such as may occur due to human error, and that moreover makes it possible to easily establish standard procedures for use.

### EXPLANATION OF REFERENCE NUMERALS

- 1: Cap for installation on spike
- 2: Spike
- 3: Shutoff clamp
- 4: Infusion tubing
- 5: Three-way stopcock
- 5a: Primary tubing upstream branch connector
- 5b: Primary tubing downstream branch connector
- 5c: Secondary tubing branch connector (needle-less port)
- 5d: Lever
- 6: Cap for installation on connector for connection with three-way stopcock
- 6a: Insertion port for connector for connection with three-way stopcock
- 6b: Hydrophobic filter
- 6c: Opening
- 6d: Lid
- 6e: Connector for connection with three-way stopcock
- 7: Cap for installation on connector for connection with intravenous drip needle
- 7a: Insertion port for connector for connection with intravenous drip needle
- 7b: Hydrophobic filter
- 7c: Opening
- 7d: Lid
- 8: Male connector
- 9: Drip chamber
- 10: Roller clamp
- 11: Filter bag
- 12: Connector for connection with intravenous drip needle
- 13: Infusion container
- 14: Physiological saline solution
- 15: Package
- 16: Illustration

## Claims

1. An infusion set comprising:
a first member constituting primary tubing in the infusion set has a first spike (2); a first spike cap (1); a three-way stopcock (5); a male connector (8); a drip chamber (9); a roller clamp (10); a first connector (12) for connection with an intravenous drip needle; a first cap (7), for installation on the first connector for connection with the intravenous drip needle, arranged at which first cap there is a first opening (7c) not allowing passage therethrough of solid or liquid but allowing passage therethrough of gas, a first hydrophobic filter (7b) being arranged at a location inward from where a tip of the first connector for connection with the intravenous drip needle is inserted at the interior of the first cap by way of a first insertion port (7a) for the first connector for connection with said intravenous drip needle, a first lid (7d) for closing said first opening being arranged at the exterior of said first opening; first infusion tubing (4); a first shutoff clamp (3) for pressing on and opening or closing a flow path within the first infusion tubing; and a filter bag (11);
a second member constituting secondary tubing in the infusion set has a second spike (2); a second spike cap (1); second infusion tubing (4); a second shutoff clamp (3) for pressing on and opening or closing a flow path within the second infusion tubing; a second connector (6e) for connection with the three-way stopcock (5); and a second cap (6), for installation on the second connector for connection with the three-way stopcock (5), arranged at which second cap there is a second opening (6c) not allowing passage therethrough of solid or liquid but allowing passage therethrough of gas, a second hydrophobic filter (6b) being arranged at a location inward from where a tip of the second connector (6e) for connection with the three-way stopcock is inserted at the interior of the second cap by way of a second insertion port (6a) for the second connector for connection with said three-way stopcock, a second lid (6d) for closing said second opening (6c) being arranged at the exterior of said second opening;
wherein, prior to use of the infusion set, the first member constituting the primary tubing and the second member constituting the secondary tubing in the infusion set are in a mutually separate state; but during use thereof, a secondary tubing branch connector (5c) at the three-way stopcock (5) of the first member constituting the primary tubing in the infusion set and the second connector (6e) that connects with the three-way stopcock at the second member constituting the secondary tubing in the infusion set are connected, causing the first member constituting the primary tubing and the second member constituting the secondary tubing in the infusion set to be in the form of a single integral unit;
wherein the first spike (2) is provided at an upstream-most location in the first member constituting the primary tubing of the infusion set, said first spike being optionally covered with the first spike cap (1), and said first spike being connected to one end of the first infusion tubing (4) equipped with the first shutoff clamp (3) for pressing on and opening or closing a flow path within the first infusion tubing, the other end of said first infusion tubing being connected to a primary tubing upstream branch connector (5a) of the three-way stopcock (5), and further connected to a primary tubing downstream branch connector (5b) of said three-way stopcock (5) there is or are optionally one or more three-way stopcocks (5), and further connected to the primary tubing downstream branch connector (5b) of the three-way stopcock (5) is one end of the first infusion tubing equipped with the first shutoff clamp (3) for pressing on and opening or closing an infusion tubing flow path, the other end of said first infusion tubing being connected to the drip chamber (9), said drip chamber being connected to one end of the first infusion tubing on which the roller clamp (10) is installed, the other end of said first infusion tubing being connected to the primary tubing upstream branch connector (5b) of the three-way stopcock (5), the primary tubing downstream branch connector (5b) of said three-way stopcock (5) being connected to one end of the first infusion tubing, the other end of said first infusion tubing being connected to the filter bag (11) and the first infusion tubing downstream therefrom, and the other end of said first infusion tubing being further connected to the primary tubing upstream branch connector (5) of the three-way stopcock (5), the primary tubing downstream branch connector (5b) of said three-way stopcock (5) being connected to one end of the first infusion tubing, the other end of said first infusion tubing being connected to the first connector (12) for connection with an intravenous drip needle, said first connector for connection with an intravenous drip needle being covered with the first cap (7), for installation on the first connector for connection with the intravenous drip needle, arranged at which first cap there is the first opening (7c) not allowing passage therethrough of solid or liquid but allowing passage therethrough of gas, the first hydrophobic filter (7b) being arranged at a location inward from where a tip of the first connector for connection with the intravenous drip needle is inserted at the interior of the first cap by way of the first insertion port (7a) for the first connector for connection with the intravenous drip needle, the first lid (7d) for closing said first opening (7c) being arranged at the exterior of said first opening; and
wherein the second spike (2) is provided at an upstream-most location in the second member constituting the secondary tubing of the infusion set, said second spike being optionally covered with the second spike cap (1), and said second spike being connected to one end of the second infusion tubing (4) equipped with the second shutoff clamp (3) for pressing on and opening or closing a flow path within the second infusion tubing, the other end of said second infusion tubing being connected to the second connector (6e) for connection with the three-way stopcock (5), the second connector (6e) for connection with said three-way stopcock (5) being covered with the second cap (6), for installation on the second connector (6e) for connection with the three-way stopcock (5), arranged at which second cap there is the second opening (6c) not allowing passage therethrough of solid or liquid but allowing passage therethrough of gas, the second hydrophobic filter (6b) being arranged at a location inward from where a tip of the second connector (6e) for connection with the three-way stopcock (5) is inserted at the interior of the second cap by way of the second insertion port (6a) for the second connector for connection with said three-way stopcock (5), the second lid (6d) for closing said second opening (6c) being arranged at the exterior of said second opening.

2. The infusion set according to claim 1, wherein the aforesaid three-way stopcock (5) at which the second infusion tubing (4) is connected to the secondary tubing branch connector (5c), said second infusion tubing (4), the second shutoff clamp (3) linked thereto, and the second cap (6) are grouped together as a single group such that one or more thereamong has the same color; and in the optional case that there are a plurality of three-way stopcocks (5) at which second spikes (1) are connected to respective secondary tubing, groups belonging to adjacent three-way stopcocks (5) respectively have different colors; and said second infusion tubing furthermore optionally has colored tape of the same color applied thereon; and any among the three-way stopcock (5), the second infusion tubing (4), the shutoff clamp (3), the second spike (1), the second cap (6) for installation on the second spike, and colored tape is furthermore optionally in a state such that a number indicating order of administration is displayed thereon.

3. The infusion set according to claim 2, wherein the aforesaid colored infusion set is further sealed within a package (15) at which package displayed in mutually offset and partially overlapping fashion on the surface thereof are a plurality of illustrations (16), the respective illustrations being respectively colored so as to have the same colors as the coloration given to groups within the infusion set sealed therewithin that comprise the aforesaid three-way stopcocks (5) at which the second infusion tubing is connected to the secondary tubing branch connectors (5c), said second infusion tubing, second shutoff clamps (3) linked thereto, and the second caps (1).

4. The infusion set according to claim 3, wherein numbers indicating order of administration are further displayed at the aforesaid illustrations.

## Patentansprüche

1. Infusionsset, umfassend:
ein erstes Element, welches einen Primärschlauch in dem Infusionsset bildet, hat einen ersten Dorn (2); eine erste Dornkappe (1); einen Dreiwegehahn (5); ein männliches Steckverbindungsstück (8); eine Tropfkammer (9); eine Rollklemme (10); ein erstes Verbindungsstück (12) zur Verbindung mit einer Tropfinfusionsnadel; eine erste Kappe (7) zur Anbringung auf dem ersten Verbindungsstück zur Verbindung mit der Tropfinfusionsnadel, wobei an der ersten Kappe eine erste Öffnung (7c), die keine Feststoffe oder Flüssigkeiten, jedoch Gas durch sie hindurchlässt, ein erster hydrophober Filter (7b), der an einem Ort einwärts von dem Ort angeordnet ist, an dem eine Spitze des ersten Verbindungsstücks zur Verbindung mit der Tropfinfusionsnadel an dem Inneren der ersten Kappe über einen ersten Einführungsanschluss (7a) für das erste Verbindungsstück zur Verbindung mit der Tropfinfusionsnadel eingeführt wird, ein erster Deckel (7d) zum Verschließen der ersten Öffnung angeordnet ist, die an der Außenseite der ersten Öffnung angeordnet ist; einen ersten Infusionsschlauch (4); eine erste Verschlussklemme (3) zum Drücken auf einen Strömungspfad innerhalb des ersten Infusionsschlauchs und zum Öffnen oder Schließen desselben; und einen Filterbeutel (11);
ein zweites Element, welches einen Sekundärschlauch in dem Infusionsset bildet, hat einen zweiten Dorn (2); eine zweite Dornkappe (1); einen zweiten Infusionsschlauch (4); eine zweite Verschlussklemme (3) zum Drücken auf einen Strömungspfad innerhalb des zweiten Infusionsschlauchs und zum Öffnen oder Schließen desselben; ein zweites Verbindungsstück (6e) zur Verbindung mit dem Dreiwegehahn (5); und eine zweite Kappe (6) zur Anbringung auf dem zweiten Verbindungsstück zur Verbindung mit dem Dreiwegehahn (5), wobei an der zweiten Kappe eine zweite Öffnung (6c), die keine Feststoffe oder Flüssigkeiten, jedoch Gas durch sie hindurchlässt, ein zweiter hydrophober Filter (6b), der an einem Ort einwärts von dem Ort angeordnet ist, an dem die Spitze des zweiten Verbindungsstücks (6e) zur Verbindung mit dem Dreiwegehahn an dem Inneren der zweiten Kappe über einen zweiten Einführungsanschluss (6a) für das zweiten Verbindungsstück zur Verbindung mit dem Dreiwegehahn eingeführt wird, angeordnet ist, wobei ein zweiter Deckel (6d) zum Verschließen der zweiten Öffnung (6c) an der Außenseite der zweiten Öffnung angeordnet ist;
wobei vor der Verwendung des Infusionssets das erste Element, das den Primärschlauch bildet, und das zweite Element, das den Sekundärschlauch in dem Infusionsset bildet, in einem voneinander getrennten Zustand sind; jedoch während deren Verwendung ein Sekundärschlauch-Zweigverbindungsstück (5c) an dem Dreiwegehahn (5) des ersten Elements, das den Primärschlauch in dem Infusionsset bildet, und das zweite Verbindungsstück (6e), das mit dem Dreiwegehahn an dem zweiten Element, das den Sekundärschlauch in dem Infusionsset bildet, eine Verbindung herstellt, verbunden sind, wodurch bewirkt wird, dass das erste Element, das den Primärschlauch in dem Infusionsset bildet, und das zweite Element, das den Sekundärschlauch in dem Infusionsset bildet, als eine einzelne integrale Einheit vorliegen;
wobei der erste Dorn (2) an einem am weitesten stromaufwärts gelegenen Ort in dem ersten Element, das den Primärschlauch in dem Infusionsset bildet, vorgesehen ist, wobei der erste Dorn wahlweise durch die erste Dornkappe (1) abgedeckt ist, und der erste Dorn mit einem Ende des ersten Infusionsschlauchs (4) verbunden ist, der mit der ersten Absperrklemme (3) zum Drücken auf einen Strömungspfad innerhalb des ersten Infusionsschlauchs und zum Öffnen oder Schließen desselben ausgestattet ist, wobei das andere Ende des ersten Infusionsschlauchs mit einem Primärschlauch-Stromaufwärts-Zweigverbindungsstück (5a) des Dreiwegehahns (5) verbunden ist, und ferner mit einem Primärschlauch-Stromabwärts-Zweigverbindungsstück (5b) des Dreiwegehahns (5) wahlweise einer oder mehrere Dreiwegehähne (5) verbunden ist bzw. sind, und ferner mit dem Primärschlauch-Stromabwärts-Zweiverbindungsstück (5b) des Dreiwegehahns (5) ein Ende des ersten Infusionsschlauchs verbunden ist, der mit der ersten Absperrklemme (3) zum Drücken auf einen Infusionsschlauch-Strömungspfad und zum Öffnen oder Schließen desselben ausgestattet ist, wobei das andere Ende des ersten Infusionsschlauchs mit der Tropfkammer (9) verbunden ist, wobei die Tropfkammer mit einem Ende des ersten Infusionsschlauchs verbunden ist, an dem die Rollklemme (10) angebracht ist, wobei das andere Ende des ersten Infusionsschlauchs mit dem Primärschlauch-Stromaufwärts-Zweigverbindungsstück (5b) des Dreiwegehahns (5) verbunden ist, wobei das Primärschlauch-Stromabwärts-Zweigverbindungsstück (5b) des Dreiwegehahns (5) mit einem Ende des ersten Infusionsschlauchs verbunden ist, wobei das andere Ende des ersten Infusionsschlauchs mit dem Filterbeutel (11) und dem ersten Infusionsschlauch stromabwärts davon verbunden ist, und das andere Ende des ersten Infusionsschlauchs ferner mit dem Primärschlauch-Stromaufwärts-Zweigverbindungsstück (5) des Dreiwegehahns (5) verbunden ist, wobei das Primärschlauch-Stromabwärts-Zweigverbindungsstück (5b) des Dreiwegehahns (5) mit einem Ende des ersten Infusionsschlauchs verbunden ist, wobei das andere Ende des ersten Infusionsschlauchs mit dem ersten Verbindungsstück (12) zur Verbindung mit einer Tropfinfusionsnadel verbunden ist, wobei das erste Verbindungsstück zur Verbindung mit einer Tropfinfusionsnadel mit der ersten Kappe (7) zur Anbringung auf dem ersten Verbindungsstück zur Verbindung mit der Tropfinfusionsnadel abgedeckt ist, wobei an der ersten Kappe die erste Öffnung (7c) angeordnet ist, die keine Feststoffe oder Flüssigkeiten, jedoch Gas durch sie hindurchlässt, wobei der erste hydrophobe Filter (7b) an einem Ort einwärts von dem Ort angeordnet ist, an dem eine Spitze des ersten Verbindungsstücks zur Verbindung mit der Tropfinfusionsnadel an dem Inneren der ersten Kappe über den ersten Einführungsanschluss (7a) für das erste Verbindungsstück zur Verbindung mit der Tropfinfusionsnadel eingeführt wird, wobei der erste Deckel (7d) zum Verschließen der ersten Öffnung (7c) an der Außenseite der ersten Öffnung angeordnet ist; und
wobei der zweite Dorn (2) an einem am weitesten stromaufwärts gelegenen Ort in dem zweiten Element, das den Sekundärschlauch des Infusionssets bildet, vorgesehen ist, wobei der zweite Dorn wahlweise mit der zweiten Dornkappe (1) abgedeckt ist, und der erste Dorn mit einem Ende des zweiten Infusionsschlauchs (4) verbunden ist, der mit der zweiten Absperrklemme (3) zum Drücken auf einen Strömungspfad innerhalb des zweiten Infusionsschlauchs und zum Öffnen oder Schließen desselben ausgestattet ist, wobei das andere Ende des zweiten Infusionsschlauchs mit dem zweiten Verbindungsstück (6e) zur Verbindung mit dem Dreiwegehahn (5) verbunden ist, wobei das zweite Verbindungsstück (6e) zur Verbindung mit dem Dreiwegehahn (5) mit der zweiten Kappe (6) zur Anbringung an dem zweiten Verbindungsstück (6e) zur Verbindung mit dem Dreiwegehahn (5) abgedeckt ist, wobei an der zweiten Kappe die zweite Öffnung (6c) angeordnet ist, die keine Feststoffe oder Flüssigkeiten, jedoch Gas durch sie hindurchlässt, wobei der zweite hydrophobe Filter (6b) an einem Ort einwärts von dem Ort angeordnet ist, an dem eine Spitze des zweiten Verbindungsstücks (6e) zur Verbindung mit dem Dreiwegehahn (5) an dem Inneren der zweiten Kappe über den zweiten Einführungsanschluss (6a) für das zweite Verbindungsstück zur Verbindung mit dem Dreiwegehahn (5) eingeführt wird, wobei der zweite Deckel (6d) zum Verschließen der zweiten Öffnung (6c) an der Außenseite der zweiten Öffnung angeordnet ist.

2. Infusionsset gemäß Anspruch 1, wobei der genannte Dreiwegehahn (5), an dem der zweite Infusionsschlauch (4) mit dem Sekundärschlauch-Zweigverbindungsstück (5c) verbunden ist, der zweite Infusionsschlauch (4), die zweite Absperrklemme (3), die damit verbunden ist, und die zweite Kappe (6) als eine einzelne Gruppe zusammen gruppiert sind, sodass eines oder mehrere Elemente davon die gleiche Farbe haben; und in dem optionalen Fall, dass es eine Mehrzahl von Dreiwegehähnen (5) gibt, an denen zweite Dorne (1) mit entsprechenden Sekundärschläuchen angeschlossen sind, Gruppen, die zu benachbarten Dreiwegehähnen (5) gehören, jeweils andere Farben haben; und auf dem zweiten Infusionsschlauch ferner wahlweise farbiges Band derselben Farbe angebracht ist; und ein beliebiges Element aus dem Dreiwegehahn (5), dem zweiten Infusionsschlauch (4), der Absperrklemme (3), dem zweiten Dorn (1), der zweiten Kappe (6) zur Anbringung auf dem zweiten Dorn und farbigem Band ferner wahlweise in einem derartigen Zustand ist, dass eine Zahl, die eine Verabreichungsreihenfolge angibt, darauf angezeigt ist.

3. Infusionsset gemäß Anspruch 2, wobei das genannte farbige Infusionsset ferner in eine Packung (15) eingesiegelt ist, wobei auf der Packung in gegenseitig abgesetzter und teilweise überlappender Art und Weise auf deren Oberfläche eine Mehrzahl von Abbildungen (16) angezeigt ist, wobei die jeweiligen Abbildungen entsprechend eingefärbt sind, sodass sie die gleiche Farbe wie die Einfärbung haben, die Gruppen innerhalb des darin eingesiegelten Infusionssets verliehen ist, die die genannten Dreiwegehähne (5) umfassen, an denen der zweite Infusionsschlauch mit den Sekundärschlauch-Zweigverbindungsstücken (5c), dem zweiten Infusionsschlauch, zweiten Absperrklemmen (3), die damit verbunden sind, und den zweiten Kappen (1) verbunden ist.

4. Infusionsset gemäß Anspruch 3, wobei Zahlen, die eine Verabreichungsreihenfolge angeben, ferner an den genannten Abbildungen angezeigt sind.

## Revendications

1. Kit de perfusion comprenant :
un premier élément, qui constitue un tube principal du kit de perfusion et qui possède une première pointe (2) ; un embout de première pointe (1) ; un robinet d'arrêt à trois voies (5) ; un connecteur mâle (8) ; une chambre de compte-gouttes (9) ; une roulette de précision (10) ; un premier connecteur (12) destiné au raccordement à une aiguille compte-gouttes intraveineuse ; un premier embout (7), pour l'installation sur le premier connecteur en vue du raccordement avec l'aiguille compte-goutte intraveineuse, au niveau du premier embout se trouvant une première ouverture (7c) qui empêche le passage d'un solide ou d'un liquide par celle-ci mais qui permet le passage d'un gaz, un premier filtre hydrophobe (7b) étant prévu à un emplacement situé vers l'intérieur et depuis lequel une extrémité du premier connecteur en vue du raccordement avec l'aiguille compte-goutte intraveineuse est insérée à l'intérieur du premier embout à l'aide d'un premier orifice d'insertion (7a) destiné au raccordement du premier connecteur avec ladite aiguille compte-goutte intraveineuse, un premier capot (7d) destiné à fermer ladite première ouverture étant prévu à l'extérieur de ladite première ouverture ; un premier tube de perfusion (4) ; une première roulette d'arrêt (3) destinée à appuyer sur et à ouvrir ou à fermer un trajet d'écoulement dans le premier tube de perfusion ; et un sachet filtrant (11) ;
un second élément, qui constitue un tube secondaire du kit de perfusion et qui possède une seconde pointe (2) ; un embout de seconde pointe (1) ; un second tube de perfusion (4) ; une seconde roulette d'arrêt (3) destinée à appuyer sur et à ouvrir ou fermer un trajet d'écoulement dans le second tube de perfusion ; un second connecteur (6e) destiné au raccordement avec le robinet d'arrêt à trois voies (5) ; et un second embout (6), pour l'installation sur le second connecteur en vue du raccordement avec le robinet d'arrêt à trois voies (5), au niveau dudit second embout se trouvant une seconde ouverture (6c) qui empêche le passage d'un solide ou d'un liquide par celle-ci mais qui permet le passage d'un gaz, un second filtre hydrophobe (6b) étant prévu à un emplacement vers l'intérieur au niveau duquel une extrémité du second connecteur (6e) en vue du raccordement avec le robinet d'arrêt à trois voies est insérée à l'intérieur du second embout à l'aide d'un second orifice d'insertion (6a) pour le second connecteur destiné au raccordement avec ledit robinet d'arrêt à trois voies, un second capot (6d) destiné à fermer ladite seconde ouverture (6c) étant prévu à l'extérieur de ladite seconde ouverture ;
dans lequel, avant l'utilisation du kit de perfusion, le premier élément qui constitue le tube principal et le second élément qui constitue le second tube du kit de perfusion sont séparés ; mais, pendant l'utilisation, un connecteur de dérivation de tube secondaire (5c) au niveau du robinet d'arrêt à trois voies (5) du premier élément qui constitue le tube principal du kit de perfusion et le second connecteur (6e) qui est relié au robinet d'arrêt à trois voies au niveau du second élément qui constitue le tube secondaire du kit de perfusion sont reliés, permettant au premier élément qui constitue le tube principal et au second élément qui constitue le tube secondaire du kit de perfusion d'être sous la forme d'une seule unité ;
dans lequel la première pointe (2) est prévue le plus en amont du premier élément qui constitue le tube principal du kit de perfusion, ladite première pointe étant éventuellement recouverte de l'embout de première pointe (1), et ladite première pointe étant reliée à une extrémité du premier tube de perfusion (4) équipée de la première roulette d'arrêt (3) afin d'appuyer sur et d'ouvrir ou de fermer un trajet d'écoulement dans le premier tube de perfusion, l'autre extrémité dudit premier tube de perfusion étant reliée à un connecteur de dérivation amont de tube principal (5a) du robinet d'arrêt à trois voies (5), et sont reliés à un connecteur de dérivation aval de tube principal (5b) dudit robinet d'arrêt à trois voies (5) un ou plusieurs robinets d'arrêt à trois voies (5) qui sont ou peuvent être présent(s), et est reliée au connecteur de dérivation aval de tube principal (5b) du robinet d'arrêt à trois voies (5) une extrémité du premier tube de perfusion équipée de la première roulette d'arrêt (3) afin d'appuyer sur et d'ouvrir ou de fermer un trajet d'écoulement de tube de perfusion, l'autre extrémité dudit premier tube de perfusion étant reliée à la chambre compte-goutte (9), ladite chambre compte-goutte étant reliée à une extrémité du premier tube de perfusion sur lequel la roulette de précision (10) est installée, l'autre extrémité dudit premier tube de perfusion étant reliée au connecteur de dérivation amont de tube principal (5b) du robinet d'arrêt à trois voies (5), le connecteur de dérivation aval de tube principal (5b) dudit robinet d'arrêt à trois voies (5) étant relié à une extrémité du premier tube de perfusion, l'autre extrémité dudit premier tube de perfusion étant reliée au sachet filtrant (11) et au premier tube de perfusion en aval de celui-ci, et l'autre extrémité dudit premier tube de perfusion étant en outre reliée au connecteur de dérivation amont de tube principal (5) du robinet d'arrêt à trois voies (5), le connecteur de dérivation aval de tube principal (5b) dudit robinet d'arrêt à trois voies (5) étant relié à une extrémité du premier tube de perfusion, l'autre extrémité dudit premier tube de perfusion étant reliée au premier connecteur (12) en vue du raccordement avec une aiguille compte-goutte intraveineuse, ledit premier connecteur destiné à être relié à une aiguille compte-goutte intraveineuse étant recouvert par le premier embout (7), en vue de l'installation sur le premier connecteur pour le raccordement avec l'aiguille compte-goutte intraveineuse, au niveau du premier embout se trouvant la première ouverture (7c) qui empêche le passage d'un solide ou d'un liquide par celle-ci mais qui permet le passage d'un gaz, le premier filtre hydrophobe (7b) étant prévu à un emplacement intérieur depuis lequel une extrémité du premier connecteur destiné au raccordement avec l'aiguille compte-goutte intraveineuse est insérée à l'intérieur du premier embout à l'aide du premier orifice d'insertion (7a) de sorte que le premier connecteur soit relié à l'aiguille compte-goutte intraveineuse, le premier capot (7d) destiné à fermer ladite première ouverture (7c) étant prévu à l'extérieur de ladite première ouverture ; et
dans lequel la seconde pointe (2) est prévue au niveau d'un emplacement le plus en amont dans le second élément qui constitue le tube secondaire du kit de perfusion, ladite seconde pointe étant éventuellement recouverte de l'embout de seconde pointe (1), et ladite seconde pointe étant reliée à une extrémité du second tube de perfusion (4) équipée de la seconde roulette d'arrêt (3) afin d'appuyer sur et d'ouvrir ou de fermer un trajet d'écoulement dans le second tube de perfusion, l'autre extrémité dudit second tube de perfusion étant reliée au second connecteur (6e) destiné au raccordement avec le robinet d'arrêt à trois voies (5), le second connecteur (6e) destiné au raccordement avec ledit robinet d'arrêt à trois voies (5) étant recouvert par le second embout (6), pour l'installation sur le second connecteur (6e) en vue du raccordement avec le robinet d'arrêt à trois voies (5), au niveau du second embout se trouvant la seconde ouverture (6c) qui empêche le passage d'un solide ou d'un liquide par celle-ci mais qui permet le passage d'un gaz, le second filtre hydrophobe (6b) étant prévu à un emplacement intérieur depuis lequel une extrémité du second connecteur (6e) destiné au raccordement avec le robinet d'arrêt à trois voies (5) est insérée à l'intérieur du second embout à l'aide du second orifice d'insertion (6a) de sorte que le second connecteur puisse être relié audit robinet d'arrêt à trois voies (5), le second capot (6d) destiné à fermer ladite seconde ouverture (6c) étant prévu à l'extérieur de ladite seconde ouverture.

2. Kit de perfusion selon la revendication 1, dans lequel ledit robinet d'arrêt à trois voies (5) au niveau duquel le second tube de perfusion (4) est relié au connecteur de dérivation de tube secondaire (5c), ledit second tube de perfusion (4), la seconde roulette d'arrêt (3) reliée à celui-ci, et le second embout (6) sont regroupés comme un seul groupe de sorte qu'un ou plusieurs d'entre eux possède la même couleur ; et, lorsqu'il y a éventuellement plusieurs robinets d'arrêt à trois voies (5) au niveau desquels des secondes pointes (1) sont reliées à des tubes secondaires respectifs, les groupes qui appartiennent aux robinets d'arrêt à trois voies adjacents (5) possèdent respectivement des couleurs différentes ; et ledit second tube de perfusion possède éventuellement en outre une bande colorée de la même couleur appliquée dessus ; et n'importe lequel du robinet d'arrêt à trois voies (5), du second tube de perfusion (4), de la roulette d'arrêt (3), de la seconde pointe (1), du second embout (6) pour l'installation sur la seconde pointe, et de la bande colorée se trouve en outre éventuellement dans un état dans lequel un chiffre qui indique l'ordre d'administration est affiché dessus.

3. Kit de perfusion selon la revendication 2, dans lequel ledit kit de perfusion coloré est en outre contenu dans un boîtier (15) sur la surface duquel se trouvent, de manière mutuellement décalée et partiellement en chevauchement, plusieurs illustrations (16), les illustrations respectives étant respectivement colorées de façon à avoir les mêmes couleurs que la coloration donnée aux groupes du kit de perfusion contenus à l'intérieur et qui comprennent lesdits robinets d'arrêt à trois voies (5) au niveau desquels les seconds tubes de perfusion sont reliés aux connecteurs de dérivation de tubes secondaires (5c), auxdits seconds tubes de perfusion, auxdites secondes roulettes d'arrêt (3) reliées à ceux-ci, et aux seconds embouts (1).

4. Kit de perfusion selon la revendication 3, dans lequel les chiffres qui indiquent l'ordre d'administration sont en outre affichés au niveau desdites illustrations.
